# EUROPEAN PATENT APPLICATION

(11) **EP 1 462 120 A1**
(43) Date of publication of application: **29.09.2004**
(21) Application number: 04005267.2
(22) Date of filing: 05.03.2004
(51) Int. Cl.: A61L 2/07, A61L 2/26, A61C 19/00, B01J 3/04, B21C 23/00

(54) **Steam sterilisation chamber for steam sterilising apparatus, in particular for sterilising dental instruments, and process for manufacturing of same**

(30) Priority: 24.03.2003 IT MI20030579
(71) Applicant: M.O.COM. S.r.L., 20124 Milano (IT)
(72) Inventor: Piazzalunga, Gianfranco, 20080 Casarile (Milano) (IT); Tosi, Daniele Marino, 27018 Vidigulfo (Pavia) (IT)
(74) Representative: Lunati, Vittoriano

(57) **Abstract**

The boiler body (1) comprises a substantially cylindrical hollow skirt (8) made by extrusion, in particular of aluminium material. The extruded skirt (8) is machined on its surface to form seats for fastening of auxiliary elements such as doors, closing means, heating elements and the like, for the boiler and/or the steriliser. The skirt (8) is closed at one end by a plate-like bottom wall applied thereto and can have longitudinal stiffening ribs or ridges on the outer surface thereof. It preferably has a cross section different from the circular section, in particular a substantially oval section, to increase the useful space for arrangement of item-holding trays (6). At least the inner surface of the skirt (8) of aluminium material is anodised to withstand contact with steam.

## Description

The invention relates to a boiler body for steam sterilising apparatus, in particular sterilisers for dental instruments, and to a process for manufacturing of same.

It is known that steam sterilisers, such as those designed for sterilisation of items or instruments in the dental field, substantially comprise a boiler, housed in a box-shaped container and internally defining a sterilisation chamber, in which the items or instruments to be sterilised are put.

The chamber is accessible through a door hinged to the boiler body at the front thereof. The items or instruments to be sterilised, as for example instrument-carrying handles (turbines, micromotors, union elbows), are disposed on trays, boxes or racks, insertable into the chamber in which they remain for the whole duration of the sterilisation cycle.

Boilers in these known sterilisers are generally made of stainless steel and have a cylindrical shape with a circular cross-section. The boiler capacity is mainly about 15-20 litres.

The techniques used for manufacturing the boiler body mainly consist in drawing stainless steel sheets or calendering stainless steel sheets that are then bent and welded with each other along a generatrix to form the cylindrical sterilisation chamber.

The technique resorting to drawing allows the chamber bottom too to be directly obtained and is therefore very advantageous.

These boilers, of relatively big usable volume, however, are not suitable for quick sterilisation cycles which may be required for example in the dental field for instrument sterilisation during the time gap between treatment of one patient and the next one. In fact the big inner volume involves relatively long times for reaching the vacuum, temperature and pressure conditions within the chamber as required for a correct sterilisation, as well as rather long times to bring back the inside of the chamber to the atmospheric temperature and pressure conditions at the end of the sterilisation cycle.

Therefore sterilising boilers of smaller sizes, 5 or 6 litres for example, have been conceived in which quicker sterilisation cycles can be carried out.

These boilers, however, have a drawback consisting in that the smaller inner volume also involves a smaller item-loading capacity for each sterilisation cycle. In particular, the usable volume is greatly reduced as the inner diameter of the boiler becomes smaller.

Therefore, the loading amount can be reduced to such an extent that a given number of items cannot be sterilised in a single sterilisation cycle, but two distinct sterilisation cycles may be required, so that the advantage resulting from a shorter duration of the cycle becomes fruitless.

In addition, reduction in the boiler diameter generally does no longer allow use of trays, racks or boxes of standard sizes but much smaller item-holding containers are required.

Under this situation the present invention mainly aims at devising a boiler body for steam-sterilising apparatus, in particular sterilisers for dental instruments, and a process for manufacturing said sterilisers, which are capable of overcoming the above mentioned drawbacks.

Accordingly, it is an important aim of the invention to devise a boiler body that is particularly adapted for sterilisers having reduced sizes and quick sterilisation cycles.

It is a further aim to provide a process that is particularly adapted for industrial implementation of boiler bodies for sterilisers of medium-small sizes.

Another aim of the invention is to provide a boiler body that, while being of small sizes, does however allow to utilise, for the sterilisation of items and instruments, item-holding trays, racks or boxes of standard sizes, as normally used for bigger sterilisers.

A still further aim of the invention consists in providing a boiler body of simplified construction that does not require use of expensive and/or complicated plants or dies and that can be obtained with working processes that are economically convenient.

The foregoing and further aims that will become more apparent in the following, are achieved by a boiler body for steam-sterilising apparatus, in particular for sterilisers for dental instruments, as well as by a process for manufacturing of same, having the features set out in claims 1 and 10, respectively.

Preferred embodiments of the invention are specified in the other claims.

Further features and advantages of the invention will be best understood from the following detailed description of some preferred embodiments of the invention, given by way of non-limiting example in the accompanying drawings, in which:
- Fig. 1 is a front view of a steam-sterilising apparatus provided with a boiler body made in accordance with the invention;
- Fig. 2 is a perspective view of a portion of the raw extruded section member from which the boiler body in Fig. 1 is obtained;
- Fig. 3 is a top perspective view of the skirt of the boiler body shown in Fig. 1, duly machined for fastening of some elements of the boiler and/or the steriliser;
- Fig. 4 is a perspective view from below of the same boiler body duly machined;
- Fig. 5 is a perspective view of the bottom wall of the boiler body shown in Fig. 1; and
- Fig. 6 is a front view of another embodiment of the boiler body in accordance with the invention.

With reference to the figures, a boiler body in accordance with the invention is generally denoted at 1 and is adapted to be installed in a steam-sterilising apparatus 2. The apparatus is shown only generally as it is of known type and substantially comprises a container 3, within which a boiler is disposed that can be opened at the front through a door 4 allowing access to the inside of the sterilisation chamber 5 defined within the boiler body 1.

The items to be sterilised such as in particular dental instruments like turbines, micromotors, union elbows and the like are placed in this chamber contained in trays 6, boxes or racks of known type, for example. Trays 6 or the like are carried for example by frame-shaped supports 7 adapted to bear one or more superposed trays 6 manually insertable into the sterilisation chamber 5.

In accordance with a feature of the invention, the boiler body 1 comprises a substantially cylindrical hollow skirt 8 consisting of an extruded element, in particular made of aluminium material.

More particularly, skirt 8 is obtained by extrusion of a section member of substantially hollow cylindrical shape, preferably made of aluminium material by means of an extruding press of known type.

Advantageously skirt 8 is formed with a plurality of ribs or ridges 9 on the outer side surface 10 thereof, which extend in the longitudinal extrusion direction of the skirt 8. Longitudinal ribs or ridges 12 may be also provided on the inner side surface 11 to form guide surfaces for trays 6 or supports 7, for example.

The raw skirt 8 is then surface machined, in particular at the front end 13 thereof and at ribs or ridges 9, by material removal for example, to form seats 14 for fastening of the boiler body 1 to the container 3 of the sterilising apparatus 2 or fastening of auxiliary boiler elements, such as door 4, door hinges, means for door closure, heating elements for the boiler, etc., that are not shown as they are of known type and are not part of the invention.

In fact, manufacture of skirt 8 by extrusion, preferably of aluminium material, allows an element of some thickness to be obtained, that can be easily worked by machine-tools so as to enable the boiler to be constructed industrially quite well.

As shown in Fig. 4, ribs 9 on the lower outer surface of skirt 8 are partly removed in the central region, where a flat portion 15 is formed that is adapted to receive the heating means of steriliser 2 in thermal-conduction contact relationship.

In the forward and rear front surfaces of ribs or ridges 9 and in the surfaces of seats 14, fastening holes 16 can be machined, in particular threaded holes for fastening of the front end of skirt 8 to the front wall 3a of the container 3 and fastening of a closing element, such as a plate-like bottom wall 17, at the rear end of skirt 8.

The bottom wall 17 has substantially the same peripheral profile as the cross section of skirt 8. It can be made by pressing of aluminium or other suitable material and can be conveniently machined to be sealingly fastened to skirt 8, by means of screws for example.

It is to be noted that the extruded skirt 8 has such a thickness as to enable threaded holes of appropriate length and width to be formed inside it, thereby allowing both other mechanical elements such as hinges, closing block, bottom, etc., and standard threaded nipples to be anchored to skirt 8 in an easy and very accurate manner without requiring further machining operations or addition of elements.

Furthermore, the front surface 13 of the extruded skirt 8 advantageously lends itself to form the rest surface for the sealing element of the steriliser door 4, by virtue of the fact that the extruded skirt is of such a thickness as to enable use of same in order to form an appropriate rest surface for the door 4 by a simple flattening operation directly carried out on the extruded body and without addition of other separate pieces.

This fact represents an important simplification in the designing, manufacturing and assembling operations and reduces errors due to couplings with other pieces such as flanges and the like.

Another important advantage of the invention resides in that through extrusion it is possible to easily obtain boiler bodies, and therefore sterilisation chambers, of different length, without particular problems, since it is sufficient to cut the extruded section member to size.

The above allows sterilisation chambers of more or less extended depth to be produced to adapt them to the market requirements without substantially being obliged to make new investments.

Advantageously, in accordance with a preferred feature of the invention, the boiler body 1 is submitted to an anodising process, in particular a so-called hard-anodising process, at least on the inner surface to allow a safe and durable resistance to contact with steam produced in chamber 5 during the sterilisation cycle.

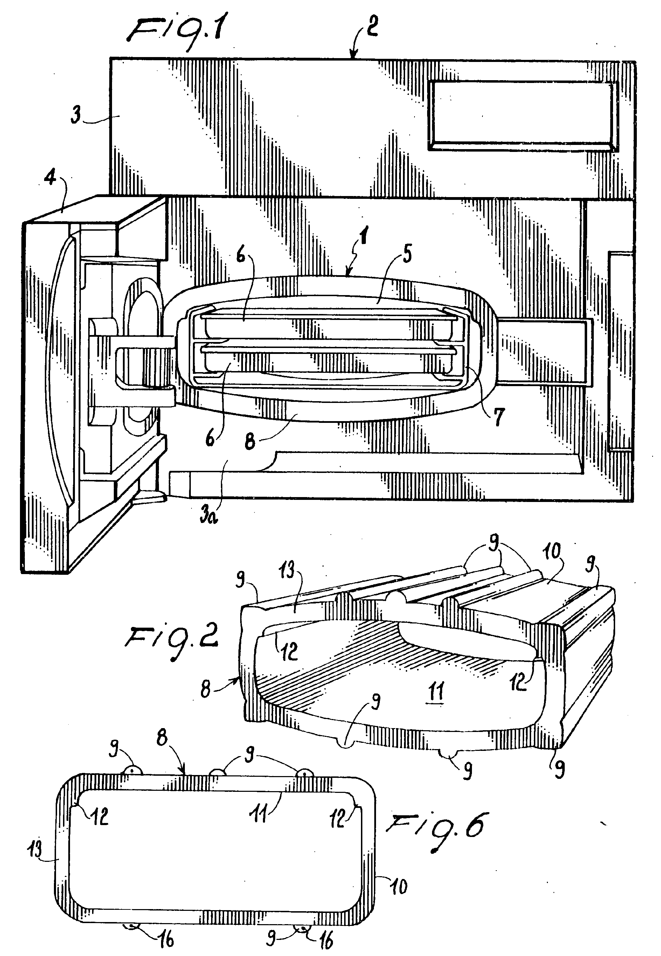

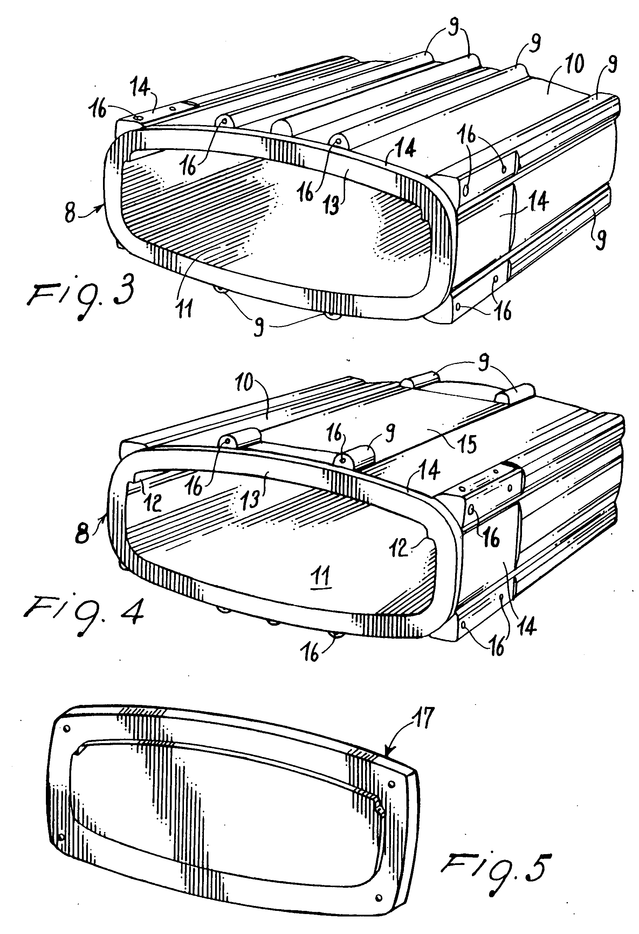

In Figs. 1-5 the boiler body 1 has a cross section of a shape different from the traditional circular shape, in particular a substantially flattened circular cross section, more particularly a substantially oval cross section.

This section shape has the advantage of enabling an optimal exploitation of the sterilisation chamber 5 because it allows to reduce the chamber height and to increase the chamber width, the chamber volume being the same, so that arrangement of superposed trays is allowed and the usable space in chamber 5 can be utilised almost completely.

In other words, this shape allows high loading volumes to be reached, while keeping the overall volume of the sterilisation chamber 5 low, 5-6 litres for example, and while speeding up the sterilisation cycles, thanks to the reduced volume and thus to the shorter time necessary for creating the vacuum, pressure and temperature conditions for sterilisation as well as for evacuating the chamber itself.

Said form also enables use of trays, boxes or racks of standard size currently used in sterilisers of bigger sizes, even in the presence of a smaller volume as compared with a chamber of circular cross section with a diameter adapted to house such standard trays or the like.

Shown in Fig. 6 by way of example is another possible cross section shape of a boiler body 1 in accordance with the invention, having a substantially rectangular profile with rounded corners. This embodiment too has the advantage of a big usable volume in the boiler and is adapted for use of trays, boxes or racks of standard size.

The cross section of the boiler body 1 could also be formed of two substantially parallel opposite sides and two arched opposite sides facing each other and connected to the substantially parallel sides.

It will be appreciated that the process for manufacturing skirt 8 by extrusion, of aluminium material in particular, practically allows accomplishment of the desired section shape within very wide limits, while avoiding construction of expensive and/or complicated dies. In addition, the wall thickness of skirt 8 can be substantially high and therefore a great sturdiness is ensured, while at the same time limiting the overall weight of the boiler body 1.

Based on the section shape, arrangement of the longitudinal ribs or ridges 9 on skirt 8 can obviously be different depending on the stresses that will arise in the particular case.

Instead of being fastened by screws or the like, the bottom wall 17 and/or the auxiliary elements of the boiler and/or steriliser could be welded to body 1, at least partly.

The described invention achieves important advantages.

In fact, it allows boiler bodies to be obtained that are particularly adapted to sterilisers having high operating speeds and therefore quick sterilisation cycles. In addition, it makes it possible to obtain a great number of cross section shapes for the boiler, and therefore particularly advantageous boiler bodies in terms of space exploitation in the sterilisation chamber, because higher loading volumes than those of chambers of conventional circular cross section can be reached, the chamber volume being the same.

The invention therefore allows production of boilers that, while having a smaller inner volume, are able to utilise item-carrying trays, racks or boxes that are presently only adapted to boilers of bigger sizes.

The invention also allows boilers of different length or depth to be easily produced with limited costs.

Furthermore, by the invention boilers of high sturdiness can be obtained, following simple and economically convenient working processes.

The invention is susceptible of modifications within the frame of the appended claims. In particular, instead of aluminium, another metallic or non-metallic material adapted for extrusion could be used.

## Claims

1. A boiler body for steam-sterilising apparatus, in particular for sterilisers (2) for dental instruments, comprising a substantially cylindrical hollow skirt (8) closed at one end by a bottom wall (17) and internally defining a sterilisation chamber (5) adapted to receive items to be sterilised, **characterised in that** at least said skirt (8) is made of an extruded element.

2. A boiler body as claimed in claim 1, wherein said extruded element is made of aluminium material.

3. A boiler body as claimed in claim 1 or 2, wherein said skirt (8) has a cross section different from a circular cross section.

4. A boiler body as claimed in one or more of claims 1, 2 or 3, wherein said skirt (8) has a substantially circular flattened cross section.

5. A boiler body as claimed in one or more of claims 1, 2 or 3, wherein said skirt (8) has a substantially oval cross section.

6. A boiler body as claimed in one or more of claims 1, 2 or 3, wherein said skirt (8) has a cross section defined by two substantially parallel opposite sides and two arched opposite sides facing each other and connected to said substantially parallel opposite sides.

7. A boiler body as claimed in one or more of the preceding claims, wherein said skirt (8) has longitudinal ribs or ridges (9) at least on the outer surface (10) thereof.

8. A boiler body as claimed in claim 2, wherein at least the inner surface (11) of said skirt (8) is made of anodised aluminium material.

9. A boiler body as claimed in one or more of the preceding claims, wherein said bottom wall (17) is made up of a plate-like element sealingly applied to said skirt (8).

10. A process for manufacturing a boiler body (1) for steam-sterilising apparatus, in particular sterilisers (2) for dental instruments, **characterised in that** in consists in extruding a section member of substantially hollow cylindrical shape and in applying, at an end of the extruded section member, a closing element adapted to form a bottom wall (17) of the boiler body (1).

11. A process as claimed in claim 10, wherein said section member is made of aluminium material.

12. A process as claimed in claim 11, wherein at least the inner surface of said extruded section member is submitted to an anodising process.

13. A process as claimed in claim 10, 11 or 12, wherein said extruded section member is further machined to form fastening seats (14) for auxiliary elements such as doors, door hinges, closing means, heating elements and the like of the boiler and/or the sterilising apparatus.
